# EUROPEAN PATENT SPECIFICATION

(11) **EP 0 536 186 B1**
(45) Date of publication and mention of the grant of the patent: **21.11.2001**
(21) Application number: 91911087.4
(22) Date of filing: 15.05.1991
(51) Int. Cl.: C12N 15/12, C12P 21/02, A61K 38/18

(54) **BONE AND CARTILAGE INDUCTIVE PROTEINS**
KNOCHEN- UND KNORPEL-BILDUNG HERVORRUFENDE PROTEINE
PROTEINES D'INDUCTION OSSEUSE ET CARTILAGINEUSE

(30) Priority: 16.05.1990 US 525357; 15.01.1991 US 641204
(43) Date of publication of application: 14.04.1993
(73) Proprietor: GENETICS INSTITUTE, INC., Cambridge, Massachusetts 02140 (US)
(72) Inventor: HEWICK, Rodney, M., Lexington, MA 02173 (US); WANG, Jack, H., Lexington, MA 02173 (US)
(74) Representative: VOSSIUS & PARTNER
(86) International application number: US9103388
(87) International publication number: WO9118098

(56) References cited:
- WO-A-89/10409
- WO-A-90/11366
- WO-A-92/07073
- PROC. NATL. ACAD. SCI. U.S.A., Vol. 86, June 1989, Washington, US, pages 4554-4558, LYONS K. et al., "Vgr-1, a Mammalian Gene Related to Xenopus Vg-1, is a Member of the Transforming Growth Factor beta Gene Superfamily".

## Description

This application is a continuation-in-part of U.S. Serial No. 07/525,357 filed May 16, 1990 and U.S. Serial No. 07/641,204 filed January 15, 1991.

The present invention relates to a family of purified proteins which may exhibit the ability to induce cartilage and/or bone formation and processes for obtaining them. These proteins may be used to induce bone and/or cartilage formation and in wound healing and tissue repair.

The invention provides a novel family of proteins termed BMP-8 proteins. Bovine and perhaps other species BMP-8 proteins are characterized by comprising at least one of the following sequences or one of the following sequences with one or more modification(s) of O-linked or N-linked glycosylation sites: and by the ability to induce the formation of cartilage and/or bone.

The BMP-8 proteins of the invention may be further characterized by an apparent molecular weight of 28,000 - 38,000 daltons as determined by sodium dodecyl sulfate polyacrylamide gel electrophoresis (SDS-PAGE). Under reducing conditions in SDS-PAGE the protein reveals a region of approximately 14,000-20,000 daltons.

The BMP-8 proteins may be further characterized by a DNA sequence encoding BMP-8 comprising at least one of the following DNA sequences

It is contemplated that the proteins of the invention are capable of stimulating, promoting, or otherwise inducing cartilage and/or bone formation.

The amino acid sequences set forth above are derived from a bovine bone preparation as further described herein. Based on knowledge of other "BMP" proteins it is expected that the human sequence is the same or homologous to the bovine sequences. The invention further includes human BMP-8 protein and the DNA encoding human BMP-8 as disclosed herein in deposit #75010.

The invention further includes methods for obtaining the DNA sequences encoding the BMP-8 proteins of the invention. This method entails utilizing the above amino acid sequences or portions thereof to design probes to screen libraries for the human gene or fragments thereof using standard techniques.

The proteins of the invention may be produced by culturing a cell transformed with a DNA sequence encoding the BMP-8 protein and recovering and purifying from the culture medium a protein characterized by comprising at least one or the following sequences or one of the following sequences with one or more modification(s) of O-linked or N-linked glycosylation sites: and by the ability to induce the formation of cartilage and/or bone

The expressed protein is isolated, recovered and purified from the culture medium. The purified expressed protein is substantially free from other proteinaceous materials with which it is co-produced, as well as from other contaminants. The recovered purified protein is contemplated to exhibit cartilage and/or bone formation activity.

It is further contemplated that the proteins of the invention demonstrate activity in the rat bone formation assay described below at a concentration of .5*µ* - 500*µ* g/gram of bone formed. It is further contemplated that these proteins demonstrate activity in this assay at a concentration of 1*µ*g - 50*µ*g/gram bone. More particularly, it is contemplated these proteins may be characterized by the ability of 1*µ*g of the protein to score at least +2 in the rat bone formation assay.

Another aspect of the invention provides pharmaceutical compositions containing a therapeutically effective amount of a protein of the invention in a pharmaceutically acceptable vehicle or carrier. The compositions of the invention may be used to induce bone and/ or cartilage formation. These compositions may also be used for wound healing and tissue repair. Further compositions of the invention may include in addition to a protein of the present invention at least one other therapeutically useful agent such as the proteins designated BMP-1, BMP-2 (also previously referred to as BMP-2A or BMP-2 Class I), BMP-3, BMP-4 (also previously referred to as BMP-2B or BMP-2 Class II) disclosed in PCT publications WO 88/00205 and WO 89/10409; and BMP-5, BMP-6, and BMP-7 disclosed in PCT publication WO 90/11366.

Other therapeutically useful agents include growth factors such as epidermal growth factor (EGF), fibroblast growth factor (FGF), and transforming growth factors (TGF-α and TGF-β). The compositions of the invention may also include an appropriate matrix, for instance, for supporting the composition and/or providing a surface for bone and/or cartilage growth. The matrix may provide slow release of the BMP protein and or the appropriate environment for presentation of the BMP protein.

The compositions may be employed in methods for treating a number of bone and/or cartilage defects, and periodontal disease. They may also be employed in methods for treating various types of wounds and in tissue repair. These methods, according to the invention, entail administering to a patient needing such bone and/or cartilage formation, wound healing or tissue repair, a therapeutically effective amount of a protein of the invention. These methods may also entail the administration of a protein (or portion thereof) of the invention in conjunction with at least one of the "BMP" proteins (or portion thereof) disclosed in the co-owned applications described above. In addition, these methods may also include the administration of a protein of the invention with other growth factors including EGF, FGF, TGF-α, and TGF-β.

Still a further aspect of the invention are DNA sequences coding for expression of a BMP-8 protein of the invention. Such sequences include a sequence of nucleotides encoding at least one of the same or substantially the same peptide sequences reported above or fragments thereof.

A further aspect of the invention provides vectors containing a DNA sequence encoding BMP-8 proteins of the invention as described above in operative association with an expression control sequence therefor. Host cells transformed with such vectors for use in producing BMP-8 proteins are also provided by the present invention. The host cells containing DNA sequences encoding BMP-8 may be employed in a novel process for producing a protein of the invention. The transformed host cells are cultured in a suitable culture medium and a protein of the invention is isolated and purified from the cells, cell lysate, or conditioned medium by conventional techniques. This process may employ a number of known cells, both prokaryotic and eukaryotic, as host cells for expression of the polypeptide.

Other aspects and advantages of the present invention will be apparent upon consideration of the following detailed description and preferred embodiments thereof.

### Brief Description of the Drawing

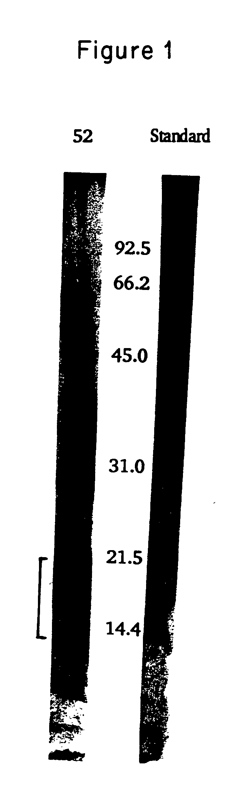

FIG. 1 illustrates an SDS-PAGE analysis of an osteoinductive fraction (28,000-38,000 daltons non-reduced) following reduction with dithiothreitol.

### Detailed Description of the Invention

A purified BMP-8 cartilage/bone protein of the present invention is characterized by comprising at least one of the following sequences or one of the following sequences with one or more modification(s) of O-linked or N-linked glycosylation sites: and by the ability to induce the formation of cartilage and/or bone.

Purified BMP-8 proteins are substantially free from proteinaceous materials with which they are co-produced as well as from other contaminants. It is contemplated that their activity may be demonstrated by activity in the rat bone formation assay as described in Example III. It is further contemplated that these proteins demonstrate activity in the assay at a concentration of .5*µ* - 500*µ*g/gram of bone formed. It is further contemplated that these proteins demonstrate activity in this assay at a concentration of 1*µ*g - 50*µ*g/gram bone. The proteins may be further characterized by the ability of 1*µ*g to score at least +2 in this assay using either the original or modified scoring method.

The proteins of the invention are further characterized by an apparent molecular weight of 28,000 - 38,000 daltons as determined by sodium dodecyl sulfate polyacrylamide gel electrophoresis (SDS-PAGE). Under reducing conditions in SDS-PAGE the protein reveals a region of approximately 14,000-20,000 daltons.

In a further aspect, the invention provides a method for obtaining the DNA sequences encoding BMP-8 bone/cartilage proteins of the invention with the above described characteristics. The method for obtaining the DNA sequences entails utilizing the amino acid sequences described above to design probes to screen libraries using standard techniques. The bovine sequence or the human gene thus identified may also be used as a probe to identify a human cell line or tissue which synthesizes the analogous cartilage/bone protein. A cDNA library is synthesized and screened with probes derived from the human or bovine coding sequences. The human sequence thus identified is transformed into a host cell, the host cell is cultured and the protein recovered, isolated and purified from the culture medium. The purified protein is predicted to exhibit cartilage and/or bone formation activity in the rat bone formation assay of Example III.

The proteins provided herein also include factors encoded by the above described sequences but into which modifications are naturally provided (e.g. allelic variations in the nucleotide sequence which may result in amino acid changes in the polypeptide) or deliberately engineered. Similarly, synthetic polypeptides which wholly or partially duplicate continuous sequences of the amino acid residues of the BMP-8 proteins are encompassed by the invention. These sequences, by virtue of sharing primary, secondary, or tertiary structural and conformational characteristics with other cartilage/bone proteins of the invention may possess bone and/or cartilage growth factor biological properties in common therewith. Thus, they may be employed as biologically active substitutes for naturally-occurring proteins in therapeutic processes.

Other specific mutations of the sequences of the proteins of the invention described herein involve modifications of O-linked or N-linked glycosylation sites. For instance, the absence of glycosylation or only partial glycosylation results from amino acid substitution or deletion at the asparagine-linked glycosylation recognition sites present in the sequences of the proteins of the invention. The asparagine-linked glycosylation recognition sites comprise tripeptide sequences which are specifically recognized by appropriate cellular glycosylation enzymes. These tripeptide sequences,are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second position) results in non-glycosylation at the modified tripeptide sequence. Expression of such altered nucleotide sequences procedures variants which are not glycosylated at that site.

The present invention also encompasses the novel DNA sequences, free of association with DNA sequences encoding other proteinaceous materials, and coding.,on expression for the proteins of the invention.

Another aspect of the present invention provides a novel method for producing the proteins of the invention. This method involves culturing a suitable cell line, which has been transformed with a DNA sequence coding for expression of a protein of the invention, under the control of known regulatory sequences. Regulatory sequences include promoter fragments, terminator fragments and other suitable sequences which direct the expression of the BMP-8 protein in an appropriate host cell. A purified BMP-8 protein of the present invention is recovered, isolated and purified from the culture medium. The purified protein is characterized by comprising at least one following sequences or one of the following sequences with one or more modification(s) of O-linked or N-linked glycosylation sites: and by the ability to induce the formation of cartilage and/or bone.

Suitable cells or cell lines may be mammalian cells, such as Chinese hamster ovary cells (CHO). The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Another suitable mammalian cell line, which is described in the accompanying examples, is the monkey COS-1 cell line. The mammalian cell line CV-1 may also be suitable. Further exemplary mammalian host cells include particularly primate cell lines and rodent cell lines, including,transformed cell lines. Normal diploid cells, cell strains derived from in vitro culture of primary tissue, as well as primary explants, are also suitable. Candidate cells may be genotypically deficient in the selection gene, or may contain a dominantly acting selection gene. Other suitable mammalian cell lines include but are not limited to , HeLa, mouse L-929 cells, 3T3 lines derived from Swiss, Balb-c or NIH mice, BHK or HaK hamster cell lines.

Bacterial cells may also be suitable hosts. For example, the various strains of E. coli (e.g., HB101, MC1061) are well-known as host cells in the field of biotechnology. Various strains of B. subtilis, Pseudomonas, other bacilli and the like may also be employed in this method.

Many strains of yeast cells known to those skilled in the art may also be available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references cited therein.

Another aspect of the present invention provides vectors for use in the method of expression of the proteins of the invention. Preferably the vectors contain the full novel BMP-8 DNA sequences described above which code for the novel cartilage/bone proteins of the invention. Additionally, the vectors also contain appropriate expression control sequences permitting expression of the protein sequences. Alternatively, vectors incorporating truncated or otherwise modified sequences as described above are also embodiments of the present invention and useful in the production of the proteins of the invention. The vectors may be employed in the method of transforming cell lines and contain selected regulatory sequences in operative association with the DNA coding sequences of the invention which are capable of directing the replication and expression thereof in selected host cells. Useful regulatory sequences for such vectors are known to those skilled in the art and may be selected depending upon the selected host cells. Such selection is routine and does not form part of the present invention. The components of the vectors, e.g. replicons, selection genes, enhancers, promoters, and the like, may be obtained from natural sources or synthesized by known procedures. See, Kaufman et al, J. Mol. Biol., 159:511-521 (1982); and Kaufman, Proc. Natl. Acad. Sci., USA, 82:689-693 (1985). Host cells transformed with such vectors and progeny thereof for use in producing cartilage/bone proteins are also provided by the invention.

A protein of the present invention, which induces cartilage and/or bone formation in circumstances where bone and/or cartilage is not normally formed, has application in the healing of bone fractures and cartilage defects in humans and other animals. Such a preparation employing a protein of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery. A protein of the invention may be used in the treatment of periodontal disease, and in other tooth repair processes. Such agents may provide an environment to attract bone-forming cells, stimulate growth of bone-forming cells or induce differentiation of progenitors of bone-forming cells. A variety of osteogenic, cartilage-inducing and bone inducing factors have been described. See, e.g. European patent applications 148,155 and 169,016 for discussions thereof.

The proteins of the invention may also be used in wound healing and related tissue repair. The types of wounds include, but are not limited to burns, incisions and ulcers. (See, e.g. PCT Publication WO84/01106 for discussion of wound healing and related tissue repair).

A further aspect of the invention includes compositions for repairing fractures and other conditions related to bone and/or cartilage defects or periodontal diseases. In addition, the invention comprises compositions for wound healing and tissue repair. Such compositions comprise a therapeutically effective amount of at least one of the proteins of the invention in admixture with a pharmaceutically acceptable vehicle, carrier or matrix. It is expected that the proteins of the invention may act in concert with or perhaps synergistically with one another or with other related proteins and growth factors. Compositions of the invention therefore comprise one or more of the proteins of the present invention. Further compositions of the invention therefore comprise a therapeutic amount of at least one protein of the invention with a therapeutic amount of at least one of the other "BMP" proteins disclosed in co-owned and co-pending. U.S. applications described above. Such compositions of the invention may comprise proteins of the invention or portions thereof in combination with the above-mentioned "BMP" proteins or portions thereof. Such combination may comprise individual molecules from each of the proteins or heteromolecules formed by portions of the respective proteins. A composition of the invention may therefore comprise a protein of the invention or a portion thereof linked with a portion of a different "BMP" as described above protein to form a heteromolecule. For example, a BMP-8 subunit may be linked to a subunit of BMP-2, BMP-3, BMP-4, BMP-5, BMP-6, BMP-7 or other BMP proteins. Such linkage may comprise disulfide bonds.

Further compositions of the invention comprise the proteins of the invention or portions thereof in combination with other agents beneficial to the treatment of the bone and/or cartilage defect, wound, or tissue in question. These agents include various growth factors such as epidermal growth factor (EGF), fibroblast growth factor (FGF), platelet derived growth factor (PDGF), transforming growth factors (TGF-α and TGF-β), and insulin-like growth factor (IGF). Portions of these agents may also be used in compositions of the invention.

The preparation and formulation of such physiologically acceptable protein compositions, having due regard to pH, isotonicity, stability and the like, is within the skill of the art. The therapeutic compositions are also presently valuable for veterinary applications due to the apparent lack of species specificity in cartilage and bone growth factor proteins. Domestic animals and thoroughbred horses in addition to humans are desired patients for such treatment with the proteins of the present invention.

The composition of the invention can be administered topically, systematically, or locally as an implant or device. When administered, the therapeutic composition for use in this invention is, of course, in a pyrogen-free, physiologically acceptable form. Further, the composition may desirably be encapsulated or injected in a viscous form for delivery to the site of cartilage and/or bone or tissue damage. Topical administration may be suitable for wound healing and tissue repair. Preferably for bone and/or cartilage formation, the composition would include a matrix capable of delivering the cartilage/bone proteins of the invention to the site of bone and/or cartilage damage, providing a structure for the developing bone and cartilage and optimally capable of being reabsorbed into the body. Such matrices may be formed of materials presently in use for other implanted medical applications.

The choice of matrix material is based on biocompatibility, biodegradability, mechanical properties, cosmetic appearance and interface properties. The particular application of the compositions of the invention will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

The dosage regimen will be determined by the attending physician considering various factors which modify the action of the proteins of the invention. Factors which may modify the action of the proteins of the invention include the amount of bone weight desired to be formed, the site of bone damage, the condition of the damaged bone, the size of a wound, type of damaged tissue, the patient's age, sex, and diet, the severity of any infection, time of administration and other clinical factors. The dosage may vary with the type of matrix used in the reconstitution and the type or types of bone and/or cartilage proteins present in the composition. The addition of other known growth factors, such as EGF, PDGF, TGF-α, TGF-β, and IGF-I to the final composition, may also effect the dosage.

Progress can be monitored by periodic assessment of cartilage and/or bone growth and/or repair. The progress can be monitored, for example, using x-rays, histomorphometric determinations and tetracycline labeling.

The following examples illustrate practice of the present invention in recovering and characterizing bovine cartilage and/or bone proteins of the invention and employing these proteins to recover the corresponding human protein or proteins and in expressing the proteins via recombinant techniques.

### EXAMPLE I

### Isolation of Bovine Cartilage/Bone Inductive Protein

Ground bovine bone powder (20-120 mesh, Colla-Tec) is prepared according to the procedures of M. R. Urist et al., Proc. Natl Acad. Sci USA, 70:3511 (1973) with elimination of some extraction steps as identified below. Ten kgs of the ground powder is demineralized in successive changes of 0.6N HCl at 4°C over a 48 hour period with vigorous stirring. The resulting suspension is extracted for 4 hours in 26 liters of 0.5M EDTA. The residue is washed two times with distilled water before its resuspension in 10 liters of 4M guanidine hydrochloride [GuCl], 1mM N-ethylmaleimide, 1mM iodoacetic acid, 1mM phenylmethylsulfonyl fluoride as described in Clin. Orthop. Rel. Res., 171: 213 (1982). After 16 to 20 hours the supernatant is removed and replaced with another 6 liters of GuCl buffer. The residue is extracted for another 8 hours. The final extraction with 6 liters of GuCl is carried out for 16 hours.

The crude GuCl extracts are combined, filtered through a Pellicon apparatus with a 0.45mM Durapore tangential flow filter packet, concentrated approximately 50 times on a Amicon RA2000 apparatus with a 10,000 molecular weight cut-off membrane, and then dialyzed in 20mM Tris, 0.05M NaCl, 6M urea (pH7.1), the starting buffer for the first column. After extensive dialysis the protein is loaded on a 2 liter DEAE cellulose column and the unbound fractions are collected.

The unbound fractions are concentrated and dialyzed against 50mM NaAc, 50mM NaCl (pH 4.6) in 6M urea. The unbound fractions are applied to a carboxymethyl cellulose column. Protein not bound to the column is removed by extensive washing with starting buffer, and the material containing protein having bone and/or cartilage formation activity as measured by the Rosen-modified Sampath - Reddi assay (described in Example III below) desorbed from the column by 50mM NaAc, 0.25mM NaCl, 6M urea (pH 4.6). The protein from this step elution is concentrated 20- to 40- fold, then dialyzed extensively against 80mM KPO₄, 6M urea (pH6.0). The sample is applied to an hydroxylapatite column (IBF) equilibrated in 80mM KPO₄, 6M urea (pH6.0) and all unbound protein is removed by washing the column with the same buffer. Protein having bone and/or cartilage formation activity is eluted with 100mM KPO₄ (pH7.4) and 6M urea.

The protein is diluted 5 fold with a 0.1875 M NaCl, 6 M urea solution to a final concentration of 20 mM KPO₄, 150 mM NaCl, 6 M urea. This material is applied to a heparin - Sepharose column equilibrated in 20mM KPO₄, 150mM NaCl, 6M urea (pH7.4). After extensive washing of the column with starting buffer, a protein with bone and/or cartilage inductive activity is eluted by 20mM KPO₄, 700mM NaCl, 6M urea (pH7.4). This fraction is concentrated 10 - 20 fold, dialyzed against 50mM NaAc, 6M urea (pH4.6), and applied to a Pharmacia MonoS HR column. The column is developed with a gradient to 1.0M NaCl, 50mM NaAc, 6M urea (pH4.6). All fractions with absorbance at 280 nm are pooled. This Mono S step is now believed to be dispensable and will be eliminated in the future. The material is applied to a 4.7 x 30 cm Waters PrepPak 500 C4 cartridge in 0.1% TFA and the column developed with a gradient to 95% acetonitrile, 0.1% TFA in 100 minutes at 45ml per minute. Fractions were assayed for cartilage and/or bone formation activity.

Aliquots of the appropriate fractions are iodinated by one of the following methods: P. J. McConahey et al, Int. Arch. Allergy, 29:185-189 (1966); A. E. Bolton et al, Biochem J., 133:529 (1973); and D. F. Bowen-Pope, J. Biol. Chem., 237:5161 (1982). The iodinated proteins present in these fractions are analyzed by SDS gel electrophoresis.

### EXAMPLE II

### Characterization of Bovine Cartilage/Bone Inductive Factor

### A. Molecular Weight

Approximately 2.5mg protein from Example I from active BMP containing fractions in 0.1% TFA and approximately 45% acetonitrile, is dried with a Savant Speed Vac concentrator and solubilized with Laemmli sample buffer, loaded onto a 12.5% polyacrylamide gel and subjected to SDS-PAGE [Laemmli, U.K. Nature, 227:680-685 (1970)] without reducing the sample with dithiothreitol. The molecular weight is determined relative to iodinated Bio-Rad molecular weight standards. Following autoradiography of the unfixed gel the approximate 28,000-38,000 dalton band is excised and the protein electrophoretically eluted from the gel [Hunkapillar et al Meth. Enzymol. 91:227-236 (1983)]. Based on similar purified bone fractions as described in the co-pending "BMP" applications described above wherein bone and/or cartilage activity is found in the approximately 28,000-38,000 region, it is inferred that this band comprises bone and/or cartilage inductive fractions.

### B. Subunit Characterization

The subunit composition of the isolated bovine bone protein is also determined. The eluted protein described above is fully reduced and alkylated in 2% SDS using iodoacetate and standard procedures. The fully reduced and alkylated sample is then further submitted to SDS-PAGE on a 12.5% gel and the resulting approximate 14,000-20,000 dalton region having a doublet/triplet appearance located by autoradiography of the unfixed gel. A silver stain [Merril et al, Science, 211 : 1437 (1981)] version of the sample is shown in FIG. 1 along with molecular weight markers. The 14,000-20,000 dalton region is indicated by the bracket. Thus the approximate 28,000-30,000 dalton protein yields a broad region of 14,000-20,000.

### EXAMPLE III

### Rosen Modified Sampath-Reddi Assay

A modified version of the rat bone formation assay described in Sampath and Reddi, Proc. Natl. Acad. Sci. U.S.A., 80:6591-6595 (1983) is used to evaluate bone and/or cartilage activity of the proteins of the invention. This modified assay is herein called the Rosen-modified Sampath-Reddi assay. The ethanol precipitation step of the Sampath-Reddi procedure is replaced by dialyzing (if the composition is a solution) or diafiltering (if the composition is a suspension) the fraction to be assayed against water. The solution or suspension is then redissolved in 0.1 % TFA, and the resulting solution added to 20mg of rat matrix. A mock rat matrix sample not treated with the protein serves as a control. This material is frozen and lyophilized and the resulting powder enclosed in #5 gelatin capsules. The capsules are implanted subcutaneously in the abdominal thoracic area of 21 - 49 day old male Long Evans rats. The implants are removed after 7 - 14 days. Half of each implant is used for alkaline phosphatase analysis [See, A. H. Reddi et al., Proc. Natl Acad Sci., 69:1601 (1972)].

The other half of each implant is fixed and processed for histological analysis. Glycolmethacrylate sections (1*µ*m) are stained with Von Kossa and acid fuschin or toluidine blue to score the amount of induced bone and cartilage formation present in each implant. The terms +1 through +5 represent the area of each histological section of an implant occupied by new bone and/or cartilage cells and newly formed bone and matrix. A score of +5 indicates that greater than 50% of the implant is new bone and/or cartilage produced as a direct result of protein in the implant. A score of +4, +3, +2 and +1 would indicate that greater than 40%, 30%, 20% and 10% respectively of the implant contains new cartilage and/or bone.

It is contemplated that the dose response nature of the cartilage and/or bone inductive protein containing samples of the matrix samples will demonstrate that the amount of bone and/or cartilage formed increases with the amount of cartilage/bone inductive protein in the sample. It is contemplated that the control samples will not result in any bone and/or cartilage formation.

As with other cartilage and/or bone inductive proteins such as the above-mentioned "BMP" proteins, the bone and/or cartilage formed is expected to be physically confined to the space occupied by the matrix. Samples are also analyzed by SDS gel electrophoresis and isoelectric focusing followed by autoradiography. The activity is correlated with the protein bands and pI. To estimate the purity of the protein in a particular fraction an extinction coefficient of 1 OD/mg-cm is used as an estimate for protein and the protein is run on SDS PAGE followed by silver staining or radioiodination and autoradiography.

### EXAMPLE IV

### Bovine Protein Composition

The gel slice of the approximate 14,000-20,000 dalton region described in Example IIB is excised and the protein electrophoretically eluted from the gel (Hunkapillar, et al., Supra.). This isolated protein sample is then depleted of SDS [Simpson, et al., Eur. J. Biochem. 165:21-29 (1987)] by being bound to a 30 x 2.1 mm Brownlee RP-18 after dilution with 5 volumes of 90% n-propanol. Protein is recovered by eluting with a step of 40% n-proponal, 0.1% TFA. The fractions containing the eluted protein peak are pooled and brought to near dryness in a savant Speed Vac concentrator. The protein is then re-solubilized with 0.1 M ammonium bicarbonate and digested with 1 *µ*g of TPCK - treated trypsin (Worthington) for 16 hours at 37°C. A second 1 *µ*g dose of trypsin was added and digestion continued for another 4 hours. The resultant digest is then subjected to RPHPLC using a C4 Vydac RPHPLC column and 0.1% TFA-water, 0.1% TFA water-acetonitrile gradient. The resultant peptide peaks were monitored by UV absorbance at 214 and 280 nm and subjected to direct amino terminal amino acid sequence analysis using an Applied Biosystems gas phase sequenator (Model 470A). Three tryptic fragments are isolated by standard procedures having the following amino acid sequence as represented by the amino acid standard three-letter symbols and where the amino acid in parentheses indicates uncertainty in the sequence:

The four amino acid sequences identified above share homology with other BMP proteins BMP-2, BMP-3, and BMP-4 disclosed in PCT published applications WO 88/00205 and WO 89/10409, BMP-5, BMP-6, and BMP-7 disclosed in USSN's 437,409, 490,033, and 438,919 filed November 15, 1989, November 15, 1989 and November 17, 1989, respectively. Specifically, the above amino acid sequence shares homology with BMP-2, BMP-3, BMP-4, BMP-5, BMP-6 and BMP-7 which contain the following human homologous sequences: The second amino acid sequence shares homology with the following human sequences of these BMP molecules:

The third amino acid sequence shares homology with the following human sequences of these BMP molecules:

The fourth amino acid sequence shares some homology (i.e. Asn-Glu-Leu-Pro-) with BMP-3, disclosed in PCT published applications WO 88/00205 and WO 89/10409.

It is contemplated that the BMP-8 proteins of the invention will be structurally similar to these BMP proteins BMP-2 through BMP-7. It is contemplated that mature BMP-8 proteins comprise a dimer of disulfide linked polypeptide subunits.

### EXAMPLE V

### Isolation of DNA

DNA sequences encoding BMP-8 proteins may be isolated using various techniques known to those skilled in the art. As described below, oligonucleotide probes may be designed on the basis of the amino acid sequence of the above-identified tryptic fragments and synthesized on an automatic DNA synthesizer. The probes may consist of pools of oligonucleotides or unique oligonucleotides designed from the tryptic sequences according to the method of R. Lathe, J. Mol. Biol. 183(1):1-12 (1985).

Based on the similarity of the three amino acid sequences described above to BMP-2 through BMP-7 it is contemplated that the BMP-8 proteins of the invention may have a structure in which amino acid sequence (3) and amino acid sequence (2) are located immediately adjacent to each other as follows:

The following four oligonucleotides are designed on the basis of the amino acid sequence of the above identified tryptic fragment [BMP-8 amino acid sequence (2) Leu-(Ser)-Ala-Thr-Ser-Val-Leu-Tyr-Tyr-Asp-Ser-Ser-Asn-Asn-Val-Ile-Leu-Arg] and synthesized on an automated DNA synthesizer.

The first 9 nucleotides of oligonucleotides #1 through #4 (underlined) contain the recognition sequence for the restriction endonuclease XbaI in order to facilitate manipulation of a specifically amplified DNA sequence encoding the BMP-8 protein and thus are not derived from the amino acid sequence (2) presented above.

The following oligonucleotide is designed on the basis of the amino acid sequence of another above identified tryptic fragment [BMP-8 amino acid sequence (3) Ala-Cys-Cys-Ala-Pro-Thr-Lys] and synthesized on an automated DNA synthesizer.

The first 8 nucleotides of oligonucleotide #5 (underlined) contain the recognition sequence for the restriction endonuclease BamHI and for reasons described above are not derived from the amino acid sequence (3).

The standard nucleotide symbols in the above identified probes are as follows: A,adenosine; C, cytidine; G, guanosine; T, thymidine; N, adenosine or cytidine or guanosine or thymidine; R, adenosine or guanosine; Y, cytidine or thymidine; and H, adenosine or cytidine or thymidine.

Oligonucleotides #4 and #5 identified above are utilized as primers to allow the amplification of a specific nucleotide sequence from bovine genomic DNA. The amplification reaction is performed as follows:

Bovine genomic DNA (source: bovine liver) is denatured at 100° C for 5 minutes and then chilled on ice before adding to a reaction mixture containing 200 *µ*M each deoxynucleotide triphosphates (dATP, dGTP, dCTP and dTTP), 10 mM Tris-HCl pH 8.3, 50mM KCl, 1.5 mM MgCl₂, 0.001% gelatin, 1.25 units Taq DNA polymerase, 100 pM oligonucleotide #4 and 100 pM oligonucleotide #5. This reaction mixture is incubated at 94° C for 2 minutes and then subjected to thermal cycling in the following manner. 1 minute at 94° C, 1 minute at 40 ° C, 1 minute at 72° C for three cycles then 1 minute at 94° C, 1 minute at 55 ° C, 1 minute at 72° C for thirty-seven cycles, followed by a 7 minute incubation at 72° C.

The DNA which is specifically amplified by this reaction is ethanol precipitated, digested with the restriction endonucleases XbaI and BamHI and subjected to agarose gel electrophoresis. A region of the gel is excised, the DNA is electroeluted and an 80 base pair product is subcloned into the plasmid vector pGEM3 between the XbaI and BamHI sites of the polylinker. DNA sequence analysis of resulting subclones indicates that the specifically amplified DNA sequence product encodes the amino acid sequences set forth in tryptic fragments (2) and (3).

The DNA sequence (SEQ ID NO: 5) and derived amino acid sequence (SEQ ID NO: 6) of this specifically amplified DNA fragment is as follows:

Nucleotides 1-24 of this sequence comprise a portion of oligonucleotide #5 and nucleotides 58-80 comprise a portion of the reverse compliment of oligonucleotide #4 utilized to perform the specific amplification reaction. Due to the function of oligonucleotides #4 and #5 in initiating the amplification reaction, they may not correspond exactly to the actual sequence encoding a BMP-8 protein and are therefore not translated in the above amino acid derivation.

The following oligonucleotide probe is designed on the basis of the bovine DNA sequence set forth above and synthesized on an automated DNA synthesizer:

This oligonucleotide probe is radioactively labeled with ³²P and employed to screen a bovine genomic library constructed in the vector λ EMBL3. 400,000 recombinants of the bovine genomic library are plated at a density of 8000 recombinants per plate on 50 plates. Duplicate nitrocellulose replicas of the recombinant bacteriophage plaques are made from these plates and amplified. The oligonucleotide probe #6 is hybridized to the amplified nitrocellulose replicas in SHB (Standard Hybridization Buffer)at 65 degrees C and washed with 1X SSC, 0.1 % SDS at 65 degrees C. Eleven positively hybridizing recombinants are obtained and are plaque purified. Bacteriophage plate stocks are made and bacteriophage DNA is isolated from each of the eleven plaque purified recombinants. The oligonucleotide hybridizing region of one of the recombinants, designated λ9800-10 is localized to a 0.4 kb PstI fragment. This fragment is subcloned into a plasmid vector (pGEM-3) and DNA sequence analysis performed. The partial DNA sequence (SEQ ID NO: 7) and derived amino acid sequence (SEQ ID NO: 8) of this region of clone λ9800-10 are shown in Table 1. The bacteriophage λ9800-10 was deposited with the American Type Culture Collection, 12301 Parklawn Drive, Rockville, MD "ATCC" under the accession #75011 on May 15, 1991. This deposit meets the requirements of the Budapest Treaty of the International Recognition of the Deposit of Microorganisms for the Purpose of Patent Procedure and Regulations thereunder.

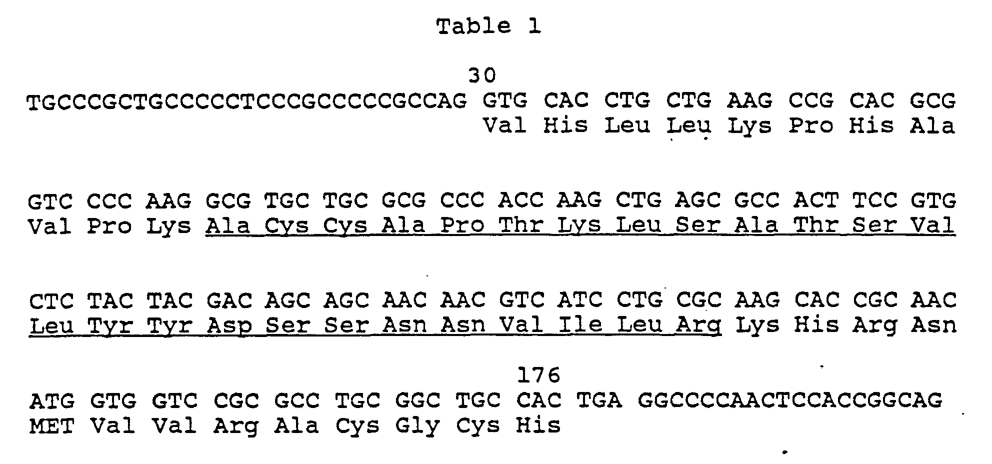

It is noted that nucleotide 95 is a "T" whereas in the amplified DNA fragment described above the corresponding nucleotide is a "C". This λ9800-10 clone encodes at least a portion of the bovine BMP-8 protein of the invention. The BMP-8 peptide sequence from this clone is 49 amino acids in length and is encoded by the DNA sequence from nucleotide 30 through nucleotide 176. The amino acid sequence corresponding to tryptic fragments (2) and (3) isolated from bovine bone 28 to 30kD material is underlined in Table 1. An in-frame stop codon (TGA) [nucleotides 177-179] indicates that this clone encodes the carboxy-terminal portion of the bovine BMP-8 protein of the invention. The nucleotides 1-29 are believed to be intron sequences based on the presence of a consensus splice site (a pyrimidine-rich stretch followed by the dinucleotide AG) as well as the lack of homology of the potentially encoded amino acids to other BMP proteins.

The following two oligonucleotides are designed on the basis of the amino sequence of tryptic fragment (1) Arg-His-Glu-Leu-Tyr-Val-Ser-Phe-Gln-Asp-Leu-Gly-Trp-Leu-Asp-Trp-Val-Ile-Ala-Pro-Gln-Gly-Tyr ((SEQ ID NO: 1) described above.

These oligonucleotides hybridize to clone λ9800-10 in SHB at 42 degrees C with washing in 5X SSC, 0.1 % SDS at 42 degrees C. A restriction fragment of clone λ9800-10 containing the hybridizing region of both oligo #7 and #8 set forth above is subcloned into a plasmid vector (pGEM-3) and DNA sequence analysis performed. The partial DNA sequence (SEQ ID NO: 9) and derived amino acid sequence (SEQ ID NO: 10) of this region of clone λ9800-10 are shown in Table 2.

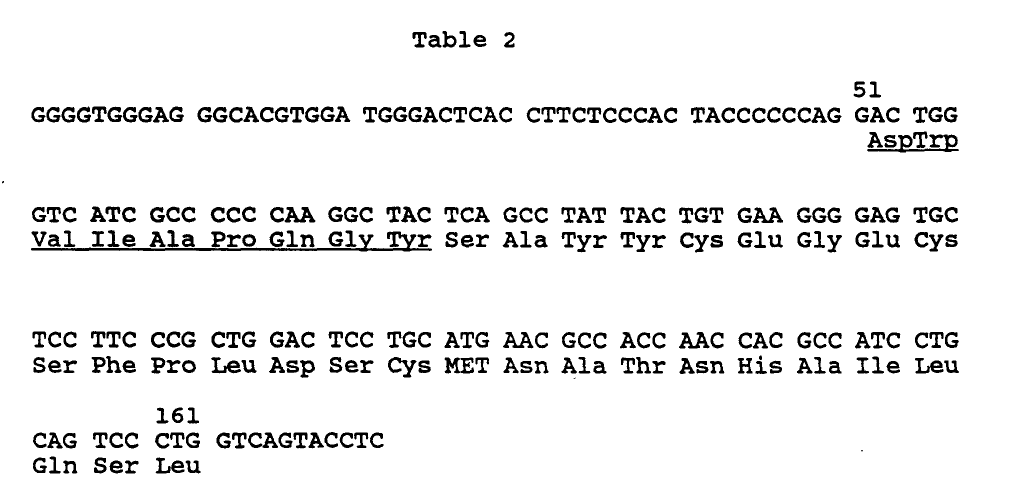

This region of clone λ9800-10 encodes another portion of the bovine BMP-8 protein of the invention. The BMP-8 peptide sequence from this clone is 37 amino acids in length and is encoded by the DNA sequence from nucleotide 51 through nucleotide 161. A portion of the amino acid sequence corresponding to tryptic fragment (1) (SEQ ID NO: 1) isolated from bovine bone 28 to 30kD material is underlined in Table 2. The nucleotides 1-50 are believed to be intron sequences based on the presence of a consensus splice site and lack of homology of the derived amino acid sequence to the remainder of the tryptic fragment (1). Similarly, the nucleotide sequences 162-172 are also believed to be intron sequences.

Another PstI restriction fragment of clone λ9800-10 is subcloned and sequenced in a similar manner to that described above. The partial DNA sequence (SEQ ID NO: 11) and derived amino acid sequence (SEQ ID NO:12) of this region of clone λ9800-10 are shown in Table 3.

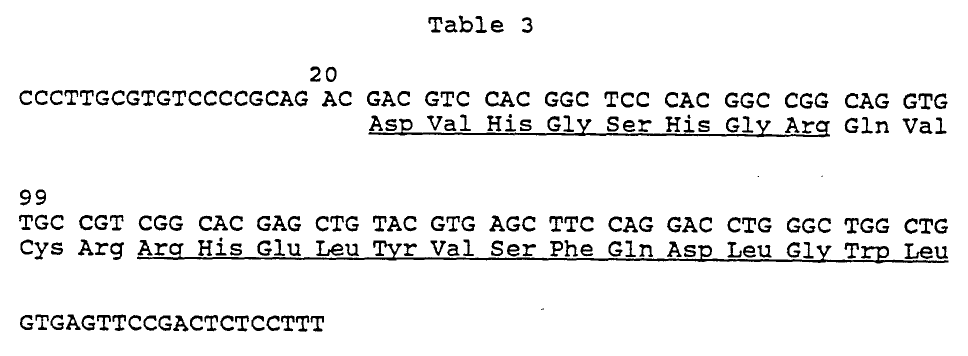

This region of clone λ9800-10 encodes another portion of the BMP-8 protein of the invention. The BMP-8 peptide sequence from this clone is 26 amino acids in length and is encoded by the DNA sequence from nucleotide 20 through nucleotide 99. The remaining portion of the amino acid sequence corresponding to tryptic fragment (1) isolated from bovine bone 28 to 30kD material is underlined in Table 3. It is also noted that this sequence encodes a peptide sequence comprising portion of the tryptic fragment (4) isolated from bovine bone 28 to 30kD material [(Thr)-Asn-Glu-Leu-Pro-Pro-(Pro)-Asn-Lys-Leu-(Pro)-Gly-Ile-Phe-Asp-Asp-Val-His-Gly-Ser-His-Gly-Arg](SEQ ID NO: 4). The amino acid sequence corresponding to this tryptic peptide is also underlined in Table 3. The nucleotide sequences 1-19 and 100-120 are believed to be intron sequences on the basis of reasons described previously.

Based on the derived amino acid sequences set forth in Tables 1, 2, and 3, the bovine BMP-8 protein of the invention is contemplated to be comprised of the amino acid sequence (SEQ ID NO: 13) present in Table 4.

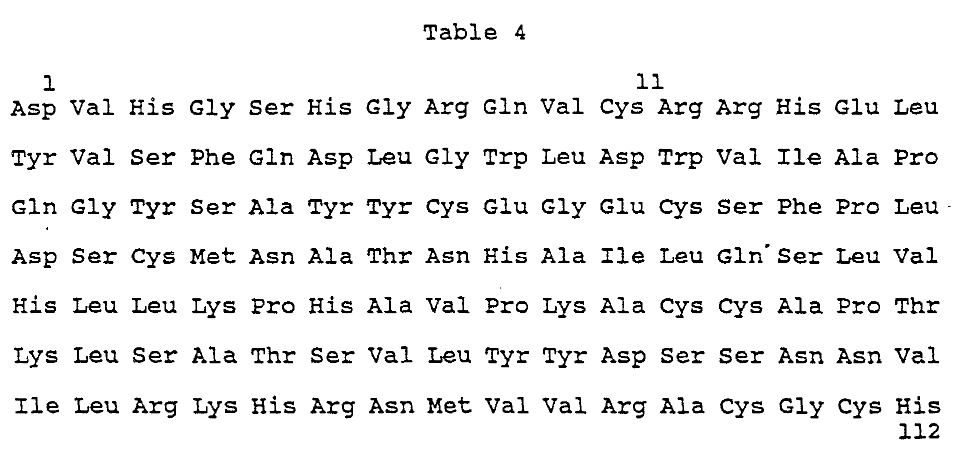

This sequence is realized to be homologous to other BMP proteins. For example, the carboxy-terminal cysteine-rich region (amino acids #11 through #112 of Table 4) demonstrates the following amino acid identities: 55% to BMP-2; 41% to BMP-3; 55% to BMP-4; 74% to BMP-5; 75% to BMP-6; and 75% to BMP-7.

### EXAMPLE V

### Human BMP-8

A 0.4 kb PstI bovine genomic BMP-8 fragment comprising the sequence set forth in Table 1 is radioactively labeled with ³²P and used as a probe to screen a human genomic library [Strategene Cloning Systems (catalog # 944201)] constructed in the vector λFIX. 1,000,000 recombinants of this human genomic library are plated at a density of 20,000 bacteriophage per plate. Duplicate nitrocellulose replicas of the recombinant bacteriophage plaques are made and hybridized to the bovine genomic probe in SHB at 65 degrees C and washed with 0.2X SSC, 0.1 % SDS at 65 degrees C. Twenty-five positives are obtained and replated for secondaries.

The following oligonucleotide probe is designed on the basis of nucleotide 57 through nucleotide 86 of the DNA sequence set forth in Table 2 and synthesized on an automated DNA synthesizer.

The following oligonucleotide probe is designed on the basis of nucleotide 20 through nucleotide 43 of the DNA sequence set forth in Table 3 and synthesized on an automated DNA synthesizer.

One set of secondary filters is hybridized to probe #9 in SHB at 65 degrees C and washed in 1X SSC, 0.1% SDS at 65 degrees C, the other set of secondary filters are hybridized to probe #10 in SHB at 50 degrees C and washed in 5X SSC, 0.1% SDS at 50 degrees C. Two clones are found to hybridize to both oligonucleotide probes. The positive hybridization of oligonucleotides #9 and #10 to these two human genomic clones indicates that they contain at least a portion of the nucleotide sequence encoding the human equivalent of the BMP-8 protein of the invention. One of these clones is designated λH8 12-1 and the bacteriophage was deposited with "ATCC" under the accession #75010 on May 15, 1991. This deposit meets the requirements of the Budapest Treaty of the International Recognition of the Deposit of Microorganisms for the Purpose of Patent Procedure and Regulations thereunder.

Once a recombinant bacteriophage containing DNA encoding a portion of the human cartilage and/or bone inductive factor molecule is obtained, the human coding sequence can be used as a probe to identify a human cell line or tissue which synthesizes the bone inductive factor. Alternatively, the bovine coding sequence can be used as a probe to identify such human cell line or tissue. Briefly described, RNA is extracted from a selected cell or tissue source and either electrophoresed on a formaldehyde agarose gel and transferred to nitrocellulose, or reacted with formaldehyde and spotted on nitrocellulose directly. The nitrocellulose is then hybridized to a probe derived from a coding sequence of the bovine or human cartilage and/or bone inductive protein. mRNA is selected by oligo (dT) cellulose chromatography and cDNA is synthesized and cloned in lambda gt10 by established techniques (Toole et al., supra).

Additional methods known to those skilled in the art may be used to isolate the human and other species' cartilage/bone proteins of the invention. The procedures described above may be employed to isolate other related proteins of interest by utilizing the bovine or human proteins as a probe source. Such other proteins may find similar utility in, inter alia, fracture repair, wound healing and tissue repair.

### EXAMPLE VI

### Expression of the Cartilage/Bone Proteins

In order to produce bovine, human or other mammalian proteins of the invention, the DNA encoding it, isolated as described above, is transferred into an appropriate expression vector and introduced into mammalian cells or other preferred eukaryotic or prokaryotic hosts by conventional genetic engineering techniques. Methods of transfection include electroporation, CaPO₄ precipitation, protoplast fusion, microinjection and lipofection. Once the host cells are transformed, stable transformants are then screened for expression of the product by standard immunological, biological or enzymatic assays. The presence of this DNA and mRNA encoding the BMP-8 polypeptides may be detected by standard procedures such as Southern and Northern blotting, high expressing cell lines are cloned or recloned at the appropriate level of selectivity to obtain a more homologous population of cells.

Selected transformed host cells are cultured and the BMP-8 proteins of the invention expressed thereby are recovered, isolated and purified. Characterization of the expressed proteins is carried out using standard techniques. For example characterization may include pulse labeling with [35 S] methionine or cysteine and analysis by polyacrylamide gel electrophoresis. The recombinantly expressed BMP-8 proteins are free of proteinaceous materials with which they are coproduced and with which they ordinarily are associated in nature, as well as from other contaminants, such as materials found in the cellular media.

It is contemplated that the preferred expression system for biologically active recombinant human proteins of the invention will be stably transformed mammalian cells. For transient expression the cell line of choices is expected to be SV40 transformed African green monkey kidney COS-1 to COS-7 which typically produce moderate amounts of the protein encoded within the plasmid for a period of 1-4 days. It is further contemplated that the preferred mammalian cells will be CHO cells.

One skilled in the art can construct mammalian expression vectors by employing the DNA sequences of the invention and known vectors, such as pCD [Okayama et al., Mol. Cell Biol., 2:161-170 (1982)] and pJL3, pJL4 [Gough et al., EMBO J., 4:645-653 (1985)]. The transformation of these vectors into appropriate host cells may result in expression of the proteins of the invention. One skilled in the art could manipulate the sequences of the invention by eliminating or replacing the mammalian regulatory sequences flanking the coding sequence with bacterial sequences to create bacterial vectors for intracellular or extracellular expression by bacterial cells. For example, the coding sequences could be further manipulated (e.g. ligated to other known linkers or modified by deleting non-coding sequences there-from or altering nucleotides therein by other known techniques). The modified coding sequence could then be inserted into a known bacterial vector using procedures such as described in T. Taniguchi et al., Proc. Natl Acad. Sci. USA, 77:5230-5233 (1980). This exemplary bacterial vector could then be transformed into bacterial host cells and a protein of the invention expressed thereby. For a strategy for producing extracellular expression of a cartilage and/or bone protein of the invention in bacterial cells., see, e.g. European patent application EPA 177,343.

Similar manipulations can be performed for the construction of an insect vector [See, e.g. procedures described in published European patent application 155,476] for expression in insect cells. A yeast vector could also be constructed employing yeast regulatory sequences for intracellular or extracellular expression of the factors of the present invention by yeast cells. [See, e.g., procedures described in published PCT application WO86/00639 and European patent application EPA 123,289].

A method for producing high levels of a protein of the invention from mammalian cells involves the construction of cells containing multiple copies of the heterologous gene encoding proteins of the invention. The heterologous gene may be linked to an amplifiable marker, e.g. the dihydrofolate reductase (DHFR) gene for which cells containing increased gene copies can be selected for propagation in increasing concentrations of methotrexate (MTX) according to the procedures of Kaufman and Sharp, J. Mol. Biol., 159:601-629 (1982). This approach can be employed with a number of different cell types. For example, a plasmid containing a DNA sequence for a protein of the invention in operative association with other plasmid sequences enabling expression thereof and the DHFR expression plasmid pAdA26SV(A)3 [Kaufman and Sharp, Mol. Cell. Biol., 2:1304 (1982)] may be co-introduced into DHFR-deficient CHO cells, DUKX-BII, by calcium phosphate coprecipitation and transfection, electroperation or protoplast fusion. DHFR expressing transformants are selected for growth in alpha media with dialyzed fetal calf serum, and subsequently selected for amplification by growth in increasing concentrations of MTX (sequential steps in 0.02, 0.2, 1.0 and 5uM MTX) as described in Kaufman et al., Mol Cell Biol., 5:1750 (1983). Transformants are cloned, and the proteins of the invention are recovered, isolated, and purified from the culture medium. Biologically active protein expression is monitored by the Rosen-modified Sampath - Reddi rat bone formation assay described above in Example III. Protein expression should increase with increasing levels of MTX resistance. Similar procedures can be followed to produce other related proteins.

### EXAMPLE VII

### Biological Activity of Expressed Cartilage/Bone Proteins

To measure the biological activity of the expressed BMP-8 proteins obtained in Example VI above, the protein may be partially purified on a Heparin Sepharose column and further purified using standard purification techniques known to those skilled in the art. For example, post transfection conditioned medium supernatant collected from the cultures may be concentrated by ultrafiltration, dialyzed and applied to a Heparin Sepharose column.

Further purification may be achieved by preparative NaDodSO₄/PAGE [Laemmli, Nature 227:680-685 (1970)]. For instance, the protein is applied to a gel. Recovery may be estimated by adding L-[³⁵S]methionine-labeled BMP protein purified over heparin-Sepharose as described above. Protein may be visualized by copper staining of an adjacent lane [Lee, et al., Anal. Biochem. 166:308-312 (1987)]. Appropriate bands are excised and extracted.

The appropriate amount of the resulting solution is mixed with 20 mg of rat matrix and then assayed for in vivo bone and/or cartilage formation activity by the Rosen-modified Sampath - Reddi assay. A mock transfection supernatant fractionation is used as a control. The implants containing rat matrix to which specific amounts of human proteins of the invention have been added are removed from rats after seven days and processed for histological evaluation. Representative sections from each implant are stained for the presence of new bone mineral with von Kossa and acid fuchsin, and for the presence of cartilage-specific matrix formation using toluidine blue. The types of cells present within the section, as well as the extent to which these cells display phenotype are evaluated and scored as described in Example III.

## Claims

1. A bone morphogenic protein (BMP-8) comprising at least one of the following sequences or one of the following sequences with one or more modification(s) of O-linked or N-linked glycosylation sites: and being further **characterized by** the ability to induce the formation of cartilage and/or bone.

2. The protein of claim 1 further **characterized by** an apparent molecular weight of 28,000-38,000 daltons as determined by SDS-PAGE under non-reducing conditions and an apparent molecular weight of about 14,000-20,000 daltons as determined by SDS-PAGE under reducing conditions.

3. A DNA sequence encoding the protein of claim 1 or 2.

4. An expression vector containing the DNA sequence of claim 3.

5. A host cell transformed with the vector of claim 4.

6. The protein of claim 1 or 2 obtainable by the steps of:
(a) culturing the host cell of claim 5; and
(b) recovering, isolating and purifying from said culture medium said protein.

7. A method for producing the protein of claim 1 or 2 said method comprising the steps of:
(a) culturing the host cell of claim 5; and
(b) recovering, isolating and purifying from said culture medium said protein.

8. A pharmaceutical composition comprising an effective amount of the protein of claim 1, 2 or 6 in admixture with a pharmaceutically acceptable carrier.

9. The pharmaceutical composition of claim 8 for bone and/or cartilage formation.

10. The pharmaceutical composition of claim 8 for wound healing and tissue repair.

11. The composition of any one of claims 8 to 10 further comprising a matrix for supporting said composition and providing a surface for bone and/or cartilage formation.

12. The composition of claim 11 wherein said matrix comprises a material selected from hydroxyapatite, collagen, polylactic acid or tricalcium phosphate.

13. A method for obtaining a DNA sequence of claim 3 **characterized in that**
(a) the amino acid sequence(s) defined in claim 1 are used to design nucleic acid probes to screen libraries; and
(b) positive clones containing DNA sequences encoding a bone morphogenic protein (BMP-8) are isolated.

14. A method for the preparation of a pharmaceutical composition comprising the combining of an effective amount of the protein of claim 1, 2 or 6 with a pharmaceutically acceptable carrier.

15. The method of claim 14 further comprising the combining with a matrix for supporting said composition and providing a matrix for bone and/or cartilage formation.

## Patentansprüche

1. Morphogenetisches Knochenprotein (BMP-8), das mindestens eine der folgenden Sequenzen, oder eine der folgenden Sequenzen mit einer oder mehreren Modifikationen der O-verknüpften oder N-verknüpften Glycosylierungsstellen umfaßt: und weiter durch die Fähigkeit charakterisiert wird, die Bildung von Knorpel und/oder Knochen zu induzieren.

2. Protein nach Anspruch 1, das weiter charakterisiert ist durch ein augenscheinliches Molekulargewicht von 28.000 - 38.000 Dalton, bestimmt durch SDS-PAGE unter nicht-reduzierenden Bedingungen, und ein augenscheinliches Molekulargewicht von 14.000 - 20.000 Dalton, bestimmt durch SDS-PAGE unter reduzierenden Bedingungen.

3. DNA-Sequenz, die das Protein nach Anspruch 1 oder 2 codiert.

4. Expressionsvektor, der die DNA-Sequenz nach Anspruch 3 enthält.

5. Wirtszelle, die mit dem Vektor nach Anspruch 4 transformiert ist.

6. Protein nach Anspruch 1 oder 2, das erhältlich ist durch die Schritte:
(a) Züchtung der Wirtszelle nach Anspruch 5; und
(b) Gewinnung, Isolierung und Aufreinigung des Proteins aus dem Kulturmedium.

7. Verfahren zur Herstellung des Proteins nach Anspruch 1 oder 2, wobei das Verfahren die Schritte umfaßt:
(a) Züchtung der Wirtszelle nach Anspruch 5; und
(b) Gewinnung, Isolierung und Aufreinigung des Proteins aus dem Kulturmedium.

8. Arzneimittel, das eine wirksame Menge des Proteins nach Anspruch 1, 2 oder 6 im Gemisch mit einem pharmazeutisch verträglichen Träger umfaßt.

9. Arzneimittel nach Anspruch 8 zur Knochen- und/oder Knorpelbildung.

10. Arzneimittel nach Anspruch 8 zur Wundheilung und Gewebereparatur.

11. Zusammensetzung nach einem der Ansprüche 8 bis 10, die weiter eine Matrix zur Unterstützung der Zusammensetzung und zur Bereitstellung einer Oberfläche für die Knochen- und/oder Knorpelbildung umfaßt.

12. Zusammensetzung nach Anspruch 11, wobei die Matrix ein Material ausgewählt aus Hydroxyapatit, Collagen, Polymilchsäure oder Tricalciumphosphat umfaßt.

13. Verfahren, um eine DNA-Sequenz nach Anspruch 3 zu erhalten, **dadurch gekennzeichnet, daß**
(a) die Aminosäuresequenz(en), definiert nach Anspruch 1, verwendet werden um Nucleinsäuresonden zur Durchmusterung von Bibliotheken zu konstruieren; und
(b) die positiven Clone, die DNA-Sequenzen enthalten, die ein morphogenetisches Knochenprotein (BMP-8) codieren, isoliert werden.

14. Verfahren zur Herstellung eines Arzneimittels, umfassend die Kombination einer wirksamen Menge des Proteins nach Anspruch 1, 2 oder 6 mit einem pharmazeutisch verträglichen Träger.

15. Verfahren nach Anspruch 14, das weiter die Kombination mit einer Matrix zur Unterstützung der Zusammensetzung und zur Bereitstellung einer Matrix zur Knochen- und/oder Knorpelbildung umfaßt.

## Revendications

1. Une protéine pour la morphogenèse osseuse (BMP-8) comprenant au moins une des séquences suivantes ou une des séquences suivantes avec une ou plusieurs modification(s) de sites de glycosylation O-liés ou N-liés : et étant en outre **caractérisée par** la capacité à induire la formation de cartilage et/ou d'os.

2. La protéine de la revendication 1, **caractérisée en outre par** une masse moléculaire apparente de 28 000 à 38 000 daltons telle que déterminée par SDS-PAGE en conditions **non réductrices**, et une masse moléculaire apparente d'environ 14 000 à 20 000 daltons telle que déterminée par SDS-PAGE en conditions **réductrices**.

3. Une séquence ADN codant la protéine de la revendication 1 ou 2.

4. Un vecteur d'expression contenant la séquence ADN de la revendication 3.

5. Une cellule hôte transformée par le vecteur de la revendication 4.

6. La protéine de la revendication 1 ou 2 susceptible d'être obtenue par les étapes de :
(a) cultiver la cellule hôte de la revendication 5 ; et
(b) récupérer, isoler et purifier ladite protéine à partir dudit milieu de culture.

7. Une méthode pour produire la protéine de la revendication 1 ou 2, ladite méthode comprenant les étapes de :
(a) cultiver la cellule hôte de la revendication 5 ; et
(b) récupérer, isoler et purifier ladite protéine à partir dudit milieu de culture.

8. Une composition pharmaceutique comprenant une quantité efficace de la protéine de la revendication 1, 2 ou 6 en mélange avec un véhicule pharmaceutiquement acceptable.

9. La composition pharmaceutique de la revendication 8 pour la formation d'os et/ou de cartilage.

10. La composition pharmaceutique de la revendication 8 pour la cicatrisation des blessures et la reconstruction des tissus.

11. La composition de l'une quelconque des revendications 8 à 10, comprenant en outre une matrice destinée à donner un support à cette composition et à fournir une surface pour la formation d'os et/ou de cartilage.

12. La composition de la revendication 11, dans laquelle ladite matrice comprend un matériau choisi parmi l'hydroxyapatite, le collagène, l'acide polylactique, ou le phosphate tricalcique.

13. Une méthode pour obtenir une séquence ADN de la revendication 3, **caractérisée en ce que**
(a) la (les) séquence(s) aminoacide(s) définies en revendication 1 sont utilisées pour construire des sondes d'acide nucléique pour cribler des banques ; et
(b) les clones positifs contenant les séquences ADN codant une protéine pour la morphogenèse osseuse (BMP-8) sont isolés.

14. Une méthode pour la préparation d'une composition pharmaceutique comprenant la combinaison d'une quantité efficace de la protéine de la revendication 1, 2 ou 6 avec un véhicule pharmaceutiquement acceptable.

15. La méthode de la revendication 14, comprenant en outre la combinaison avec une matrice destinée à donner un support à ladite composition et à fournir une matrice pour la formation d'os et/ou de cartilage.


### SEQUENCE LISTING

(1) GENERAL INFORMATION:
   (i) APPLICANT:
      Hewick, Rodney M.
      Wang, Jack H.
   (ii) TITLE OF INVENTION: Bone and Cartilage Inductive Proteins
   (iii) NUMBER OF SEQUENCES: 13
   (iv) CORRESPONDENCE ADDRESS:
      (A) ADDRESSEE: Legal Affairs, Genetics Institute, Inc.
      (B) STREET: 87 CambridgePark Drive
      (C) CITY: Cambridge
      (D) STATE: MA
      (E) COUNTRY: USA
      (F) ZIP: 02140
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE: 15-MAY-1991
      (C) CLASSIFICATION:
   (viii) ATTORNEY/AGENT INFORMATION:
      (A) NAME: Kapinos, Ellen J.
      (B) REGISTRATION NUMBER: 32,245
      (C) REFERENCE/DOCKET NUMBER: GI5182X-PCT
   (ix) TELECOMMUNICATION INFORMATION:
      (A) TELEPHONE: 617-876-1170
      (B) TELEFAX: 617-876-5851
(2) INFORMATION FOR SEQ ID NO:1:
   (i) SEQUENCE CHARACTERISTICS:
      (A) LENGTH: 23 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (vi) ORIGINAL SOURCE:
      (F) TISSUE TYPE: Bone
   (xi) SEQUENCE DESCRIPTION: SEQ ID NO:1:
   Arg His Glu Leu Tyr Val Ser Phe Gln Asp Leu Gly Trp Leu Asp Trp
   1               5                   10                  15
   Val Ile Ala Pro Gln Gly Tyr
               20
(2) INFORMATION FOR SEQ ID NO:2:
   (i) SEQUENCE CHARACTERISTICS:
      (A) LENGTH: 18 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE:
      (A) ORGANISM: Bos taurus
      (F) TISSUE TYPE: Bone
   (xi) SEQUENCE DESCRIPTION: SEQ ID NO:2:
   Leu Ser Ala Thr Ser Val Leu Tyr Tyr Asp Ser Ser Asn Asn Val Ile
   1               5                   10                  15
   Leu Arg
(2) INFORMATION FOR SEQ ID NO:3:
   (i) SEQUENCE CHARACTERISTICS:
      (A) LENGTH: 7 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (vi) ORIGINAL SOURCE:
      (A) ORGANISM: Bos taurus
      (F) TISSUE TYPE: Bone
   (xi) SEQUENCE DESCRIPTION: SEQ ID NO:3:
   Ala Cys Cys Ala Pro Thr Lys
   1               5
(2) INFORMATION FOR SEQ ID NO:4:
   (i) SEQUENCE CHARACTERISTICS:
      (A) LENGTH: 23 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (vi) ORIGINAL SOURCE:
      (A) ORGANISM: Bos taurus
      (F) TISSUE TYPE: Bone
   (xi) SEQUENCE DESCRIPTION: SEQ ID NO:4:
   Thr Asn Glu Leu Pro Pro Pro Asn Lys Leu Pro Gly Ile Phe Asp Asp
   1               5                   10                  15
   Val His Gly Ser His Gly Arg
               20
(2) INFORMATION FOR SEQ ID NO:5:
   (i) SEQUENCE CHARACTERISTICS:
      (A) LENGTH: 80 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bos taurus
   (vii) IMMEDIATE SOURCE:
      (B) CLONE: acc30
   (viii) POSITION IN GENOME:
      (C) UNITS: bp
   (ix) FEATURE:
      (A) NAME/KEY: CDS
      (B) LOCATION: 25..57
   (xi) SEQUENCE DESCRIPTION: SEQ ID NO:5:
   GGATCCGCGT GCTGTGCTCC GACC AAG CTG AGC GCC ACC TCC GTG CTC TAC      51
                              Lys Leu Ser Ala Thr Ser Val Leu Tyr
                               1               5
   TAC GAC AGCAGCAACA ATGTAATTCT AGA                                   80
   Tyr Asp
    10
(2) INFORMATION FOR SEQ ID NO:6:
   (i) SEQUENCE CHARACTERISTICS:
      (A) LENGTH: 11 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Lys Leu Ser Ala Thr Ser Val Leu Tyr Tyr Asp
    1               5                   10
(2) INFORMATION FOR SEQ ID NO:7:
   (i) SEQUENCE CHARACTERISTICS:
      (A) LENGTH: 199 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Bos taurus
   (vii) IMMEDIATE SOURCE:
      (A) LIBRARY: Bovine genomic
      (B) CLONE: Lambda 9800-10
   (viii) POSITION IN GENOME:
      (C) UNITS: bp
   (ix) FEATURE:
      (A) NAME/KEY: exon
      (B) LOCATION: 30..199
   (ix) FEATURE:
      (A) NAME/KEY: intron
      (B) LOCATION: 1..29
   (ix) FEATURE:
      (A) NAME/KEY: CDS
      (B) LOCATION: 30..179
   (xi) SEQUENCE DESCRIPTION: SEQ ID NO:7:
   TGCCCGCTGC CCCCTCCCGC CCCCGCCAG GTG CAC CTG CTG AAG CCG CAC GCG      53
                                   Val His Leu Leu Lys Pro His Ala
                                    1               5
   GTC CCC AAG GCG TGC TGC GCG CCC ACC AAG CTG AGC GCC ACT TCC GTG     101
   Val Pro Lys Ala Cys Cys Ala Pro Thr Lys Leu Ser Ala Thr Ser Val
        10                  15                  20
   CTC TAC TAC GAC AGC AGC AAC AAC GTC ATC CTG CGC AAG CAC CGC AAC     149
   Leu Tyr Tyr Asp Ser Ser Asn Asn Val Ile Leu Arg Lys His Arg Asn
    25                  30                  35                  40
   ATG GTG GTC CGC GCC TGC GGC TGC CAC TGAGGCCCCA ACTCCACCGG           196
   Met Val Val Arg Ala Cys Gly Cys His
                    45                  50
   CAG                                                                 199
(2) INFORMATION FOR SEQ ID NO:8:
   (i) SEQUENCE CHARACTERISTICS:
      (A) LENGTH: 49 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Val His Leu Leu Lys Pro His Ala Val Pro Lys Ala Cys Cys Ala Pro
    1               5                  10                  15
   Thr Lys Leu Ser Ala Thr Ser Val Leu Tyr Tyr Asp Ser Ser Asn Asn
               20                  25                  30
   Val Ile Leu Arg Lys His Arg Asn Met Val Val Arg Ala Cys Gly Cys
           35                  40                  45
   His
(2) INFORMATION FOR SEQ ID NO:9:
   (i) SEQUENCE CHARACTERISTICS:
      (A) LENGTH: 172 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Bos taurus
   (vii) IMMEDIATE SOURCE:
      (A) LIBRARY: Bovine genomic
      (B) CLONE: Lambda 9800-10
   (viii) POSITION IN GENOME:
      (C) UNITS: bp
   (ix) FEATURE:
      (A) NAME/KEY: exon
      (B) LOCATION: 51..161
   (ix) FEATURE:
      (A) NAME/KEY: intron
      (B) LOCATION: 1..50
   (ix) FEATURE:
      (A) NAME/KEY: intron
      (B) LOCATION: 162..172
   (ix) FEATURE:
      (A) NAME/KEY: CDS
      (B) LOCATION: 51..161
   (xi) SEQUENCE DESCRIPTION: SEQ ID NO:9:
   GGGGTGGGAG GGCACGTGGA TGGGACTCAC CTTCTCCCAC TACCCCCCAG GAC TGG         56
                                                         Asp Trp
                                                           1
   GTC ATC GCC CCC CAA GGC TAC TCA GCC TAT TAC TGT GAA GGG GAG TGC        104
   Val Ile Ala Pro Gln Gly Tyr Ser Ala Tyr Tyr Cys Glu Gly Glu Cys
             5                  10                  15
   TCC TTC CCG CTG GAC TCC TGC ATG AAC GCC ACC AAC CAC GCC ATC CTG        152
   Ser Phe Pro Leu Asp Ser Cys Met Asn Ala Thr Asn His Ala Ile Leu
        20                  25                  30
   CAG TCC CTG GTCAGTACCT C                                               172
   Gln Ser Leu
    35
(2) INFORMATION FOR SEQ ID NO:10:
   (i) SEQUENCE CHARACTERISTICS:
      (A) LENGTH: 37 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Asp Trp Val Ile Ala Pro Gln Gly Tyr Ser Ala Tyr Tyr Cys Glu Gly
     1               5                  10                  15
   Glu Cys Ser Phe Pro Leu Asp Ser Cys Met Asn Ala Thr Asn His Ala
               20                  25                  30
   Ile Leu Gln Ser Leu
           35
(2) INFORMATION FOR SEQ ID NO:11:
   (i) SEQUENCE CHARACTERISTICS:
      (A) LENGTH: 119 base pairs
      (B) TYPE: nucleic acid
      (C) STRANDEDNESS: double
      (D) TOPOLOGY: linear
   (ii) MOLECULE TYPE: DNA (genomic)
   iii) HYPOTHETICAL: NO
   (vi) ORIGINAL SOURCE:
      (A) ORGANISM: Bos taurus
   vii) IMMEDIATE SOURCE:
      (A) LIBRARY: Bovine genous.
      (B) CLONE: Lambda 9800-10
   iii) POSITION IN GENOME:
      (C) UNITS: bp
   (ix) FEATURE:
      (A) NAME/KEY: exon
      (B) LOCATION: 20..99
   (ix) FEATURE:
      (A) NAME/KEY: intron
      (B) LOCATION: 1..19
   (ix) FEATURE:
      (A) NAME/KEY: intron
      (B) LOCATION: 100..119
   (ix) FEATURE:
      (A) NAME/KEY: CDS
      (B) LOCATION: 22..99
   (xi) SEQUENCE DESCRIPTION: SEQ ID NO:11:

### INFORMATION FOR SEQ ID NO:12:

(i) SEQUENCE CHARACTERISTICS:
   (A) LENGTH: 26 amino acids
   (B) TYPE: amino acid
   (D) TOPOLOGY: linear
(ii) MOLECULE TYPE: protein
(xi) SEQUENCE DESCRIPTION: SEQ ID NO:12:
   Asp Val His Gly Ser His Gly Arg Gln Val Cys Arg Arg His Glu Leu
   Tyr Val Ser Phe Gln Asp Leu Gly Trp Leu
               20                  25

### INFORMATION FOR SEQ ID NO:13:

(i) SEQUENCE CHARACTERISTICS:
   (A) LENGTH: 112 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(vi) ORIGINAL SOURCE:
   (A) ORGANISM: Bos taurus
(xi) SEQUENCE DESCRIPTION: SEQ ID NO:13:
   Asp Val His Gly Ser His Gly Arg Gln Val Cys Arg Arg His Glu Leu
   1               5                   10                  15
   Tyr Val Ser Phe Gln Asp Leu Gly Trp Leu Asp Trp Val Ile Ala Pro
               20                  25                  30
   Gln Gly Tyr Ser Ala Tyr Tyr Cys Glu Gly Glu Cys Ser Phe Pro Leu
           35                  40                  45
   Asp Ser Cys Met Asn Ala Thr Asn His Ala Ile Leu Gln Ser Leu Val
       50                  55                  60
   His Leu Leu Lys Pro His Ala Val Pro Lys Ala Cys Cys Ala Pro Thr
   65                  70                  75                      80
   Lys Leu Ser Ala Thr Ser Val Leu Tyr Tyr Asp Ser Ser Asn Asn Val
                   85                  90                  95
   Ile Leu Arg Lys His Arg Asn Met Val Val Arg Ala Cys Gly Cys His
               100                 105                 110